# EUROPÄISCHE PATENTSCHRIFT

(11) **EP 1 860 086 B1**
(45) Veröffentlichungstag und Bekanntmachung des Hinweises auf die Patenterteilung: **14.05.2014**
(21) Anmeldenummer: 07107211.0
(22) Anmeldetag: 30.04.2007
(51) Int. Cl.: C07C 2/46, C07C 13/277, C09K 21/14

(54) **Verfahren zur Herstellung von Cyclododecatrien**
Method for manufacturing cyclododecatries
Procédé destiné à la fabrication de cyclododecatriènes

(30) Priorität: 10.05.2006 DE 102006022014
(43) Veröffentlichungstag der Anmeldung: 28.11.2007
(73) Patentinhaber: Evonik Degussa GmbH, 45128 Essen (DE)
(72) Erfinder: Herwig, Jürgen, 46569, Hünxe (DE); Brügging, Wilhelm, 45721, Haltern am See (DE); Roos, Martin, 45721, Haltern am See (DE); Wilczok, Norbert, 45481, Mülheim (DE)

(56) Entgegenhaltungen:
- EP-A1- 1 069 098
- DE-A1- 1 768 067
- DE-A1- 2 026 043
- DE-B- 1 140 569
- GB-A- 928 812
- GB-A- 1 095 835
- US-A- 3 007 974
- US-A- 3 243 467
- US-A- 3 546 309
- US-A- 3 849 371
- US-A- 4 151 316

## Beschreibung

Die vorliegende Erfindung betrifft ein kontinuierliches und diskontinuierliches Verfahren zur Herstellung cyclischer Butadien-Trimere, d. h. Cyclododecatrien-(1,5,9) (CDT) unter Verwendung eines Katalysatorsystems enthaltend Nickel und/oder Titan. Des Weiteren umfasst die vorliegende Erfindung das zuvor genannte Verfahren, bei dem zusätzlich Cyclooctadien (COD) und/oder Vinylcyclohexen (VCH) aus der Reaktionsmischung isoliert werden kann, wobei in diesem Fall bevorzugt ein Nickelenthaltendes Katalysatorsystem verwendet wird. Die vorliegende Erfindung umfasst zudem Stoffgemische mit einem bestimmten Verhältnis von CDT zu C₁₆, C₂₀, C₂₄-Kohlenwasserstoffen sowie Polymeren mit Molgewichten bis 5000. Weiterhin sind umfasst Stoffgemische mit Hauptbestandteil CDT und einem sehr niedrigen Anteil an Chlorcyclododecatrien (CI-CDT. Besagte Stoffgemische sind nach dem erfindungsgemäßen Verfahren erhältlich.

Eine große Zahl an Patenten und Publikationen befassen sich mit Verfahren bzw. Versuchen zur Herstellung von cyclischen Di- und Trimeren konjugierter Diene, insbesondere Butadien.

Die Bildung von Cyclododecatrien in Gegenwart eines Titan-Katalysators wurde zum Beispiel in der JP-A-2003064011 beschrieben. Dabei wurde die Reaktion unter Verwendung eines Katalysatorsystems aus Titantetrachlorid, 4,4'-Dichlorbenzophenon, Dimethylsulfoxid und Diethylaluminiumsesquichlorid bei einer Temperatur von 40°C durchgeführt. Nach beendeter Reaktion wurde die Mischung mit MeONa/MeOH versetzt und mit wässrigem Trinatriumcitrat gewaschen, um das Titan und Aluminium aus der organischen Reaktionsmischung zu entfernen. In JP-A-02083339 wird ein ähnliches Verfahren, ebenfalls unter Einsatz von DMSO, beschrieben. Die Gegenwart des hochsiedenden DMSO als Additiv ist nachteilig für einen industriellen Prozess, da es aus der Reaktionsmischung wieder entfernt werden muss.

FR-A-1393071 beschreibt die Bildung von CDT mit Titan und Aluminium als Katalysatorsystem. Als Titankatalysator wurde Ti(OR)₄ verwendet, wobei R ein aliphatischer C₃ - C₄-Alkylrest ist, als Aluminiumkatalysator wurde AlR'X₂ oder AI&lR'₂X verwendet, wobei R' ein geradkettiger oder verzweigter C₁ - C₁₈ Alkylrest oder ein C₁ - C₆-Cycloalkyl oder ein C₁ - C₁₀ Aralkylrest und X gleich Cl oder Br ist. Die in der FR1393071 beschriebene Reaktion benötigt sehr lange Reaktionszeiten von 18 h und ist daher für industrielle Anwendungen nicht geeignet. Ferner werden in der französischen Patentschrift keine Ausbeuten angegeben.

In der US 3,499,049 wird eine Methode zur Beschleunigung der katalytischen Trimerisierung von Butadien durch Zusatz von Wasser zur Reaktionsmischung beschrieben. Dieses Verfahren weist den Nachteil auf, dass die Menge an unerwünschten Nebenprodukten zu hoch ist. Insbesondere beim kontinuierlichen Betrieb sind die in der US 3,499,049 erzielten Ausbeuten an CDT von 83 % bzw. 62 % für eine industrielle Anwendung nicht ausreichend.

In der GB 928,812 wird ebenfalls ein Verfahren zur Herstellung von Cyclododecatrien unter Verwendung spezieller Katalysatoren, enthaltend eine semi-polare Doppelbindung im Molekül, beschrieben. Die besten Ergebnisse werden dabei mit DMSO erzielt, was wie zuvor bereits ausgeführt zu Nachteilen bei der Aufarbeitung der Reaktionsmischung führt.

Die DE 1140569 offenbart die Bildung von Dimeren und Trimeren von 1,3-Diolefinen mittels Nickel- oder Cobaltkatalysatorsystemen. Die Katalysatorsysteme enthalten zudem organometallische Verbindungen sowie Verbindungen mit Elektronendonoreigenschaften. Das Verfahren gemäß DE 11 40 569 benötigt den Einsatz von absolutierten Lösungsmitteln, was mit erheblichem technischen Aufwand und somit ökonomischen Nachteilen verbunden ist.

Bei der großtechnischen Trimerisierung von Butadien zu Cyclododecatrien (CDT) werden homogene Katalysatoren benutzt, wobei die Reaktion in einem kontinuierlichen Prozess in einem oder mehreren gerührten Kesseln durchgeführt wird. Dabei werden Teile der Reaktionsmischung kontinuierlich aus der Reaktionsmischung entnommen. Während der Aufarbeitung wird unreagiertes Ausgangsmaterial zurückgewonnen und dem Kreislauf zusammen mit frischem Butadien erneut zugeführt. Teile des Katalysators werden bei der Entnahme ebenfalls dem Reaktionsgemisch entzogen. Dadurch sinkt die Konzentration des Katalysators in der Reaktionsmischung und muss durch frischen Katalysator ersetzt werden um die Katalysatorkonzentration konstant zu halten.

Vor der Aufarbeitung des aus dem Reaktor entnommenen Materials muss der entnommene Katalysator zerstört werden. Eine Vielzahl polarer Lösungsmittel werden hierzu eingesetzt. Neben Wasser, benutzt Ube Industries z. B. Ammoniumhydroxid-Lösungen (JP-A-05-070377, JP 06-25438). Verschiedene Alkohole können ebenfalls benutzt werden (JP-A-07-625439, JP 07-625396). Insbesondere Methanol (JP-A-07-442496) und Methanol/HCI (DE-A-19 42 729) kommen bevorzugt zum Einsatz.

Die Zersetzung des Katalysators kann auch mittels Aceton (JP-A-04-301345) oder mittels einer Suspension von Calciumoxid in Wasser (NL-A-6 603 264) durchgeführt werden. Ube Industries berichtete zudem, dass die Ausbeute an CDT sinkt, wenn Wasser zur Zersetzung des Katalysators verwendet wird.

Ausgehend vom zuvor genannten Stand der Technik war es daher die Aufgabe der vorliegenden Erfindung, ein Verfahren zur Herstellung von Cyclododecatrien (CDT) mit hohen Ausbeuten und niedriger Menge an polymeren Nebenprodukten bereitzustellen. Eine weitere Aufgabe bestand darin, ein Verfahren bereitzustellen, bei dem die Menge an C₈- Dimeren niedrig ist. Eine weitere Aufgabe bestand schließlich darin, ein Verfahren bereitzustellen, das es insbesondere bei Nickel-katalysierten Systemen erlaubt, neben hohen Mengen an CDT auch Cyclooctadien zu isolieren.

Diese und weitere nicht explizit genannte Aufgaben sowie deren Lösung ergeben sich durch die nachfolgende Beschreibung sowie die Beispiele und Ansprüche.

Es wurde nun überraschend gefunden, dass Übergangsmetallkomplexe des Nickels und/oder des Titans Butadien mit hoher Selektivität zu CDT trimerisieren können. Um diese hohe Selektivität erreichen zu können, ist es notwendig, dass eine Verbindung enthaltend ein Element der 5. Hauptgruppe des Periodensystems und ein geeignetes Lösungsmittelsystem verwendet werden. Ferner wurde gefunden, dass die Reaktionstemperatur im Falle von Nickel-katalysierten Systemen unterhalb von 140 °C und im Falle von Titan-katalysierten Systemen unterhalb von 80 °C liegen sollte.

Gegenstand der Erfindung ist daher ein Verfahren zur Herstellung von CDT aus Butadien in Gegenwart eines Katalysatorsystems, welches durch die Ansprüche 1 bis 15 definiert und durch die nachfolgende Beschreibung näher spezifiziert ist. Gegenstand der vorliegenden Erfindung ist insbesondere ein kontinuierliches und/oder diskontinuierliches Verfahren zur Herstellung von Cyclododecatrien durch Umsetzung von Butadien in Gegenwart eines Lösungsmittels, zumindest eines Katalysatorsystems enthaltend Nickel und/oder Titan sowie zumindest einer Organometallverbindung zu einem Rohcyclododecatrien,
dadurch gekennzeichnet,
dass
- eine Verbindung enthaltend zumindest ein Element der 5. Hauptgruppe des Periodensystems der Elemente zum Katalysatorsystem zugegeben wird,
- das Lösungsmittel vor der Zugabe der Katalysatorkomponente 10 - 1000 ppm einer polaren Komponente der allgemeinen Formel HO-R, wobei R ausgewählt ist aus der Gruppe, die aus verzweigtem und unverzweigtem C₁ - C₁₈-Alkyl, C₁ - C₁₈-Cycloalkyl, C₁ - C₁₈-Aryl, C₁ - C₁₈-Aralkyl und H besteht, enthält, und
- dass die Reaktionstemperatur im Falle eines Nickel-enthaltenden Katalysatorsystems kleiner gleich 140 °C und im Falle eines Titan-enthaltenden Katalysatorsystems kleiner gleich 80 °C beträgt.

Ebenfalls Gegenstand der vorliegenden Erfindung ist ein Verfahren, bei dem neben CDT auch COD und/oder VCH aus der Reaktionsmischung isoliert werden kann.

Weiterhin Gegenstand der Erfindung sind Stoffgemische erhältlich nach dem erfindungsgemäßen Verfahren, dadurch gekennzeichnet, dass das Verhältnis von Cyclododecatrien zu höheren Oligomeren, z. B. C₁₆, C₂₀, C₂₄-Kohlenwasserstoffen, - ermittelt mittels Gaschromatographie (DB1-Säule) - und/oder Polymeren mit Molgewichten bis 5000 größer gleich 10 : 1, bevorzugt größer als 15 : 1, besonders bevorzugt größer als 20 zu 1 ist. Weiterhin bevorzugt ist das Verhältnis von Cyclododecatrien zu höheren Oligomerenkleiner als 60 : 1 und besonders bevorzugt kleiner als 50 : 1.

Ebenfalls Gegenstand der vorliegenden Erfindung sind Stoffgemische, dadurch gekennzeichnet, dass die Menge an Chlorcyclododecatrien im Rohcyclododecatrien und/oder im aufgereinigten Cyclododecatrien maximal 100 ppm, bevorzugt 0,01 - 80 ppm, besonders bevorzugt 0,1 bis 70 ppm, ganz besonders bevorzugt 1 bis 50 und 1 bis 30 ppm beträgt.

Diese erfindungsgemäßen Stoffgemische können im Falle von Nickel als Katalysator bevorzugt trans,trans,trans-CDT und im Falle von Titan als Katalysator bevorzugt cis, trans, trans-CDT enthalten.

Wie im Nachfolgenden bestätigt werden wird, ermöglicht das erfindungsgemäße Verfahren die Herstellung von Trimeren des Butadiens, insbesondere Cyclododecatrien-(1,5,9) (CDT) mit hoher Selektivität und hohen Ausbeuten, wobei sehr kurze Reaktionszeiten benötigt werden. Weiterhin konnte durch das erfindungsgemäße Verfahren die Menge an polymeren Nebenprodukten reduziert werden. So führt das erfindungsgemäße Verfahren zu Produktgemischen, in denen das Verhältnis von CDT zu höheren Oligomeren und/oder zu Polymeren optimiert ist. Schließlich ist das erfindungsgemäße Verfahren geeignet Roh-CDT-Gemische bzw. aufgereinigtes CDT mit einem Anteil an Cl-CDT von weniger als 100 ppm herzustellen.

Ohne an eine bestimmte Theorie gebunden zu sein, lassen sich diese überraschenden und vorteilhaften Effekte durch ein synergetisches Zusammenwirken der Verbindung enthaltend ein Element der 5. Hauptgruppe des Periodensystems, der Katalysatoren und der ROH-Komponente erklären. Insbesondere durch die Zugabe einer Verbindung enthaltend ein Element der 5. Hauptgruppe des Periodensystems wird deutlich weniger an polymeren Nebenprodukten sowie an Cl-CDT gebildet, wobei gleichzeitig die Raum-Zeit-Ausbeute und die Selektivität der Reaktion sehr hoch ist.

Ausgangsmaterialien für die Katalysatorsysteme des erfindungsgemäßen Verfahrens sind bevorzugt kommerziell erhältliche Nickel(11)- und/oder Titan (IV)-Verbindungen. Besonders bevorzugt TiX₄, mit X = F, Cl, Br, I oder Gemischen davon. Ganz besonders bevorzugt sind Nickelacetylacetonat und Titantetrachlorid.

Die Reaktion wird durchgeführt bei Katalysatorkonzentrationen von 0.01 bis 40 mmol/l, bevorzugt 0.05 bis 10 mmol/l basierend auf Nickel bzw. Titan.

Die organometallischen Verbindungen beinhalten zumindest ein Element der 1. bis 3. Hauptgruppe des Periodensystems der Elemente, bevorzugt Aluminium. Besonders bevorzugt sind Ethoxydiethylaluminium und Ethylaluminiumsesquichlorid

Das Verhältnis von Organometallverbindung zum Nickel-enthaltenden Katalysator wird derart gewählt, dass das molare Verhältnis von Nickel zur Organometallverbindung 1 : 3 bis 1 :10, bevorzugt 1 : 3 bis 1 : 6 beträgt. Die Reaktionstemperatur beträgt kleiner gleich 140 °C, bevorzugt 60 bis 140 °C, besonders bevorzugt 60 - 120 °C.

Im Falle von Titan-katalysierten Reaktionen beträgt das molare Verhältnis Titan zu Organometallverbindung 1 : 10 bis 1 :60, bevorzugt 1 : 10 bis 1 :40. Die Reaktionstemperatur beträgt kleiner gleich 80 °C, bevorzugt 20 bis 80 °C, besonders bevorzugt 30 - 75 °C und ganz besonders bevorzugt 30 bis 70 °C.

Die Verbindungen enthaltend zumindest ein Element der 5. Hauptgruppe des Periodensystems der Elemente welche Teil des katalytischen Systems sind enthalten bevorzugt ein oder mehrere Stickstoff- oder Phosphoratome. Besonders bevorzugt sind Ammoniak, Amine, Pyridine und Pyridone. Ganz besonders bevorzugt sind Ammoniak sowie primäre und sekundäre Amine wie z. B. C₁- bis C₈-Alkyl- und Dialkylamine. Die Verbindungen enthaltend zumindest ein Element der 5. Hauptgruppe des Periodensystems der Elemente können als Reinstoff oder in Form von organischen oder wässrigen Lösungen zugesetzt werden. Die Konzentration des Element der 5. Hauptgruppe des Periodensystems der Elemente beträgt bevorzugt 10 - 200 ppm, besonders bevorzugt 10 - 100 ppm, ganz besonders bevorzugt 10 - 90 ppm und insbesondere 30 - 90 ppm.

Die im erfindungsgemäßen Verfahren benutzten Lösungsmittel umfassen gesättigte und ungesättigte Lösungsmittel, unpolare aprotische Lösungsmittel, aliphatische und aromatische Kohlenwasserstoffe sowie Mischungen davon. Nicht limitierende Beispiele dafür sind Toluol, Benzol, Xylol, Hexane, Oktane, Vinylcyclohexen, Cyclohexane, Cyclooctane, Cyclooctadiene, Coyclododecane, Cyclododecatrien und Mischungen davon. Das Lösungsmittel hat bevorzugt eine Konzentration von 1 bis 99, besonders bevorzugt 5 bis 95 Gewichtsprozent in der Mischung am Ende der Reaktion oder während der Reaktion, wenn die Reaktion kontinuierlich durchgeführt wird. Das Lösungsmittel muss eine geringe Menge einer polaren Komponente der allgemeinen Formel HO-R enthalten, wobei R ausgewählt ist aus der Gruppe, die aus verzweigtem und unverzweigtem C₁ - C₁₈-Alkyl, C₁ - C₁₈-Cycloalkyl, C₁ - C₁₈-Aryl, C₁ - C₁₈-Aralkyl und H besteht, wobei die Kohlenstoffatome der Alkyl-, Cycloalkyl-, Aryl- und Aralkylreste durch ein Heteroatom, insbesondere O, N oder S ersetzt sein können bzw. wobei die Kohlenstoffatome Hydroxylgruppen, Aminogruppen und/oder Halogenatome tragen können. Besonders bevorzugt ist R ausgewählt aus der Gruppe, die aus Methyl, Ethyl, Propyl, Isopropyl, Butyl, Isobutyl, Tertbutyl und H besteht. Bevorzugt sind im Lösungsmittel 10 bis 500 ppm und 15 bis 250 ppm der polaren Komponente enthalten.

Das erfindungsgemäße Verfahren kann in Druckbereichen von 1 - 20 bar, bevorzugt 1 bis 10 bar betrieben werden. Der Betriebsdruck kann durch die Reaktionstemperatur und/oder Aufpressen von Inertgasen, bevorzugt Stickstoff, eingestellt werden.

Das erfindungsgemäße Verfahren kann kontinuierlich oder diskontinuierlich betrieben werden, wobei die Zugabe der einzelnen Komponenten bevorzugt in der folgenden Reihenfolge erfolgt:
- zunächst das Lösungsmittel inklusive polarer Komponente, danach zumindest eine Organometallverbindung, danach zumindest eine Titan- und/oder Nickel-enthaltende Verbindung, danach zumindest eine Verbindung enthaltend ein Element aus der 5. Hauptgruppe des Periodensystems und danach Butadien.
- zunächst das Lösungsmittel inklusive polarer Komponente, danach zumindest eine Titan- und/oder Nickel-enthaltende Verbindung, danach zumindest eine Organometallverbindung, danach zumindest eine Verbindung enthaltend ein Element aus der 5. Hauptgruppe des Periodensystems und danach Butadien.
- zunächst das Lösungsmittel inklusive polarer Komponente, danach zumindest eine Verbindung enthaltend ein Element aus der 5. Hauptgruppe des Periodensystems, danach zumindest eine Titan- und/oder Nickel-enthaltende Verbindung, danach zumindest eine Organometallverbindung, und danach Butadien.

Die Zugabe der einzelnen Komponenten kann mit und ohne zeitliche Verzögerung erfolgen. Es ist möglich, alle Komponenten in kurzer Zeit zuzugeben und anschließend nachzurühren, bis die Reaktion beendet ist. Es ist jedoch auch möglich, die einzelnen Komponenten über einen längeren Zeitraum zuzugeben wodurch eine kürzere Nachrührzeit benötigt wird. Kombinationen der beiden Ausführungsformen sind ebenfalls möglich. Das Nachrühren erfolgt bevorzugt bei der gleichen Temperatur, bei der die einzelnen Komponenten zugegeben wurden. Bevorzugt wird das Butadien so zugegeben, dass die Temperatur konstant auf einen bestimmten Wert gehalten wird. Die Reaktion wird bevorzugt fortgeführt, bis > 90 %, besonders bevorzugt > 95 % des Butadiens - nach gaschromatographischer Analyse - umgesetzt sind.

Anstelle zu rühren kann jede dem Fachmann bekannte Art des Durchmischens angewendet werden, z. B. mittels Umwelzpumpen oder anderer Arten von Strömungsdurchmischungen.

Das erfindungsgemäße CDT bzw. die erfindungsgemäßen Stoffgemische enthaltend CDT werden bevorzugt zu Lactamen, z. B. Laurinlactam, zu Polyamiden, z. B. Polyamid 12, oder zu Dicarbonsäuren, z. B. Dodecandisäure weiterverarbeitet oder in Duftstoffen und Flammschutzmitteln eingesetzt

Die nachfolgenden Beispiele dienen zur näheren Erläuterung der Erfindung ohne diese in irgend einer Weise einzuschränken.

### Beispiele:

### Vergleichsbeispiel 1:

In einer dreistufigen Rührkesselkaskade (10 Liter Volumen je Reaktor) wird die Umsetzung von Butadien zum Cyclododecatrien durch geführt. Dabei wird die Temperatur über die Kühlung der Doppelmäntel die Temperatur auf 60 °C gehalten. Reaktor 1 und 2 sind zusätzlich über externe Kühlkreisläufe in der Lage, die Reaktionswärme abzuführen. Der Druck im Reaktor 3 wird über ein Ventil auf 2 bis 3 bar geregelt, die Drücke in den vorgeschalteten Reaktor sind geringfügig höher. Im stationären Stand bestehen die Zuläufe zu Reaktor 1 aus 1,5 kg/h 1,3-Butadien, 0,5 kg/h Benzol, 0,34 g/h TiC1₄ (gelöst in Benzol) und 7,9 g/h Ethylaluminiumsesquichlorid (EASC) (gelöst in Benzol). Butadien und Benzol wurden vorab mit Molsieb getrocknet, so dass sich in Reaktor 1 eine Wassergehalt von etwa 15 ppm einstellt.
Der Umsatz bzgl. Butadien am Ende der Kaskade beträgt mehr als 95 %.
Die Selektivität zum CDT beträgt 87,2 %, zu den C₈-Isomeren 5,4 %, zu höheren Oligomeren (C₁₆, C₂₀ und C₂₄) 3 % und zu Polybutadien von 4,4 %.
Nach destillativer Aufarbeitung zu einem CDT mit einer Reinheit größer gleich 99 % enthält das Produkt 250 ppm Chlorid (gebunden als Monochlor-CDT).

### Vergleichsbeispiel 2:

Der Versuch wurde wie in Vergleichsbeispiel 1 durchgeführt, jedoch wird über die Konditionierung von Butadien und Benzol mit Wasser die Feuchte in Reaktor auf 110 ppm eingestellt.
Der Umsatz bzgl. Butadien am Ende der Kaskade beträgt mehr als 99 %.
Die Selektivität zum CDT beträgt 90,2 %, zu den C₈-Isomeren 3,9 %, zu höheren Oligomeren 2,8 % und zu Polybutadien von 3,1 %.
Nach destillativer Aufarbeitung zu einem CDT mit einer Reinheit größer gleich 99 % enthält das Produkt 230 ppm Chlorid (gebunden als Monochlor-CDT).

### Vergleichsbeispiel 3:

Der Versuch wurde wie in Vergleichsbeispiel 1 durchgeführt, wobei jedoch wasserfrei gearbeitet wurde. Über eine separate Dosierung wird ein Ammoniakgehalt von 85 ppm eingestellt.
Der Umsatz bzgl. Butadien am Ende der Kaskade beträgt mehr als 99 %.
Die Selektivität zum CDT beträgt 89,8 %, zu den C₈-Isomeren 4,6 %, zu höheren Oligomeren 2,1 % und zu Polybutadien von 3,5 %.
Nach destillativer Aufarbeitung zu einem CDT mit einer Reinheit größer gleich 99 % enthält das Produkt 60 ppm Chlorid (gebunden als Monochlor-CDT).

### Beispiel 1 (erfindungsgemäß):

Der Versuch wurde wie in Vergleichsbeispiel 1 durchgeführt, jedoch wird über die Konditionierung von Butadien und Benzol mit Wasser die Feuchte im Reaktor 1 auf 115 ppm eingestellt. Zusätzlich wird über eine separate Dosierung ein Ammoniakgehalt von 80 ppm eingestellt.
Der Umsatz bzgl. Butadien am Ende der Kaskade beträgt mehr als 99 %.
Die Selektivität zum CDT beträgt 93,7 %, zu den C₈-Isomeren 2,9 %, zu höheren Oligomeren 1,1 % und zu Polybutadien von 2,3 %.
Nach destillativer Aufarbeitung zu einem CDT mit einer Reinheit größer gleich 99 % enthält das Produkt weniger als 10 ppm Chlorid (gebunden als Monochlor-CDT).

### Beispiel 2 (erfindungsgemäß):

Der Versuch wurde grundsätzlich wie in Beispiel 1 durchgeführt, in Reaktor 1 wird eine Feuchte von 105 ppm und ein Ammoniakgehalt von 80 ppm eingestellt. Es wird jedoch auf den Betrieb der Rührer verzichtet, d. h. die Durchmischung in Reaktor 1 und 2 erfolgt lediglich über die Pumpenkreisläufe.
Der Umsatz bzgl. Butadien am Ende der Kaskade beträgt mehr als 98,5 %.
Die Selektivität zum CDT beträgt 92,2 %, zu den C8-Isomeren 3,1 %, zu höheren Oligomeren 1,7 % und zu Polybutadien von 3 %.
Nach destillativer Aufarbeitung zu einem CDT mit einer Reinheit größer gleich 99 % enthält das Produkt weniger als 10 ppm Chlorid (gebunden als Monochlor-CDT).

### Beispiel 3 (erfindungsgemäß):

Der Versuch wurde grundsätzlich wie in Beispiel 1 durchgeführt, in Reaktor 1 wird eine Feuchte von 120 ppm und ein Ammoniakgehalt von 75 ppm eingestellt. Es wird jedoch anstelle des Benzols als Lösungsmittel ein Gemisch aus COD und VCH (50 : 50) eingesetzt.
Der Umsatz bzgl. Butadien am Ende der Kaskade beträgt mehr als 99 %.
Die Selektivität zum CDT beträgt 93,3 %, zu den C₈-Isomeren 3,2 %, zu höheren Oligomeren 0,9 % und zu Polybutadien von 2,6 %.
Nach destillativer Aufarbeitung zu einem CDT mit einer Reinheit größer gleich 99 % enthält das Produkt etwa 30 ppm Chlorid (gebunden als Monochlor-CDT).

### Beispiel 4 (erfindungsgemäß):

Der Versuch wurde grundsätzlich wie in Beispiel 1 durchgeführt, in Reaktor 1 wird eine Feuchte von 110 ppm und ein Ammoniakgehalt von 85 ppm eingestellt. Durch eine verbesserte Kühlung wird der Reaktor 1 bei 46 °C und Reaktor 2 bei 53 °C gehalten.
Der Umsatz bzgl. Butadien am Ende der Kaskade beträgt mehr als 98 %.
Die Selektivität zum CDT beträgt 94,2 %, zu den C₈-Isomeren 2,8 %, zu höheren Oligomeren 1,2 % und zu Polybutadien von 1,8 %.
Nach destillativer Aufarbeitung zu einem CDT mit einer Reinheit größer gleich 99 % enthält das Produkt weniger als 10 ppm Chlorid (gebunden als Monochlor-CDT).

## Patentansprüche

1. Ein kontinuierliches und/oder diskontinuierliches Verfahren zur Herstellung von Cyclododecatrien durch Umsetzung von Butadien in Gegenwart eines Lösungsmittels, zumindest eines Katalysatorsystems enthaltend Nickel und/oder Titan sowie zumindest einer Organometallverbindung zu einem Rohcyclododecatrien,
**dadurch gekennzeichnet,**
**dass**
- eine Verbindung enthaltend zumindest ein Element der 5. Hauptgruppe des Periodensystems der Elemente zum Katalysatorsystem zugegeben wird,
- das Lösungsmittel vor der Zugabe der Katalysatorkomponente 10 - 1000 ppm einer polaren Komponente der allgemeinen Formel HO-R enthält, wobei R ausgewählt ist aus der Gruppe, die aus verzweigtem und unverzweigtem C₁ - C₁₈-Alkyl, C₁ - C₁₈-Cycloalkyl, C₁ - C₁₈-Aryl, C₁ - C₁₈-Aralkyl und H besteht, und
- **dass** die Reaktionstemperatur im Falle eines Nickel-enthaltenden Katalysatorsystems maximal 140 °C und im Falle eines Titan-enthaltenden Katalysatorsystems maximal 80 °C beträgt.

2. Verfahren nach Anspruch 1,
**dadurch gekennzeichnet,**
**dass** das Katalysatorsystem Nickelacetylacetonat enthält.

3. Verfahren nach Anspruch 1,
**dadurch gekennzeichnet,**
**dass** das Katalysatorsystem TiX₄, mit X = F, Cl, Br, I oder Gemischen davon, enthält.

4. Verfahren nach einem der Ansprüche 1 bis 3,
**dadurch gekennzeichnet,**
**dass** die Reaktion im Falle eines Nickel enthaltenden Katalysatorsystems bei 60 bis 120 °C und im Falle eines Titan enthaltenden Katalysatorsystems bei 30 bis 75 °C durchgeführt wird.

5. Verfahren nach einem der Ansprüche 1 bis 4,
**dadurch gekennzeichnet,**
**dass** die Komponente enthaltend ein Element der 5. Hauptgruppe des Periodensystems zumindest ein Stickstoffatom enthält.

6. Verfahren nach Anspruch 5,
**dadurch gekennzeichnet,**
**dass** ein primäres und/oder sekundäres und/oder tertiäres Amin und/oder Ammoniak und/oder ein Pyridin und/oder ein Pyridon verwendet wird.

7. Verfahren nach einem der Ansprüche 1 bis 6,
**dadurch gekennzeichnet,**
**dass** ein aromatisches oder aliphatische Lösungsmittel oder eine Mischung davon verwendet wird.

8. Verfahren nach einem der Ansprüche 1 bis 7,
**dadurch gekennzeichnet,**
**dass** das Katalysatorsystem eine Konzentration in der Reaktionsmischung von 0.01 bis 40 mmol/l an Nickel oder Titan aufweist.

9. Verfahren nach einem der Ansprüche 1 bis 8,
**dadurch gekennzeichnet**,
zunächst das Lösungsmittel einschließlich polarer Komponente, danach zumindest eine Organometallverbindung, danach zumindest eine Titan- und/oder Nickel-enthaltende Verbindung, danach zumindest eine Verbindung enthaltend ein Element aus der 5. Hauptgruppe des Periodensystems und danach Butadien zugegeben wird.

10. Verfahren nach einem der Ansprüche 1 bis 8,
**dadurch gekennzeichnet,**
**dass** zunächst das Lösungsmittel einschließlich polarer Komponente, danach zumindest eine Titan- und/oder Nickel-enthaltende Verbindung, danach zumindest eine Organometallverbindung, danach zumindest eine Verbindung enthaltend ein Element aus der 5. Hauptgruppe des Periodensystems und danach Butadien zugegeben wird.

11. Verfahren nach einem der Ansprüche 1 bis 10,
**dadurch gekennzeichnet,**
**dass** es sich bei der Organometallverbindung um eine Organoaluminiumverbindung handelt.

12. Verfahren nach Anspruch 11,
**dadurch gekennzeichnet,**
**dass** es sich bei der Organometallverbindung um Ethoxydiethylaluminium oder Ethylaluminiumsesquichlorid handelt.

13. Verfahren nach einem der Ansprüche 11 bis 12,
**dadurch gekennzeichnet,**
**dass** das Katalysatorsystem Nickel enthält und dass das molare Verhältnis zwischen Nickel und Aluminium von 1 : 3 bis 1 : 6 beträgt

14. Verfahren nach einem der Ansprüche 11 bis 12,
**dadurch gekennzeichnet,**
**dass** das Katalysatorsystem Titan enthält und dass das molare Verhältnis zwischen Titan und Aluminium von 1 : 10 bis 1 : 60 beträgt

15. Verfahren nach einem der Ansprüche 1 bis 14,
**dadurch gekennzeichnet,**
**dass** Cycloocta-1,5-dien (COD) und/oder Vinylcyclohexen (VCH) isoliert werden.

16. Verfahren nach einem der vorhergehenden Ansprüche,
**dadurch gekennzeichnet,**
**dass** das Verhältnis von Cyclododecatrien zu höheren Oligomeren mindestens 10 : 1, bevorzugt größer als 15 : 1, besonders bevorzugt größer als 20 : 1 ist.

17. Verfahren nach einem der vorhergehenden Ansprüche,
**dadurch gekennzeichnet,**
**dass** die Menge an Chlorcyclododecatrien im Roh-Cyclododecatrien und/oder im aufgereinigten Cyclododecatrien maximal 100 ppm, bevorzugt 0,01 - 80 ppm, besonders bevorzugt 0,1 bis 70 ppm, ganz besonders bevorzugt 1 bis 50 und 1 bis 30 ppm beträgt.

## Claims

1. A continuous and/or discontinuous process for preparing cyclododecatriene by reaction of butadiene in the presence of a solvent, at least one catalyst system containing nickel and/or titanium and at least one organometallic compound to form a crude cyclododecatriene,
wherein
- a compound containing at least one element of main group 5 of the Periodic Table of the Elements is added to the catalyst system,
- the solvent contains, before the addition of the catalyst component, 10-1000 ppm of a polar component of the general formula HO-R, where R is selected from the group consisting of branched and unbranched C₁-C₁₈-alkyl, C₁-C₁₈-cycloalkyl, C₁-C₁₈-aryl, C₁-C₁₈-aralkyl and H, and
- the reaction temperature is less than or equal to 140°C in the case of a nickel-containing catalyst system and less than or equal to 80°C in the case of a titanium-containing catalyst system.

2. A process as claimed in claim 1,
wherein
the catalyst system contains nickel acetylacetonate.

3. A process as claimed in claim 1,
wherein
the catalyst system contains TiX4, where X = F, Cl, Br, I or a mixture thereof.

4. A process as claimed in any one of claims 1 to 3,
wherein
the reaction is carried out at from 60 to 120°C in the case of a nickel-containing catalyst system and at from 30 to 75°C in the case of a titanium-containing catalyst system.

5. A process as claimed in any one of claims 1 to 4,
wherein
the component containing an element of main group 5 of the Periodic Table contains at least one nitrogen atom.

6. A process as claimed in claim 5,
wherein
a primary and/or secondary and/or tertiary amine and/or ammonia and/or a pyridine and/or a pyridone is used.

7. A process as claimed in any one of claims 1 to 6,
wherein
an aromatic or aliphatic solvent or a mixture thereof is used.

8. A process as claimed in any one of claims 1 to 7,
wherein
the catalyst system has a concentration in the reaction mixture of from 0.01 to 40 mmol/l of nickel or titanium.

9. A process as claimed in any one of claims 1 to 8,
wherein
firstly the solvent including polar component, then at least one organometallic compound, then at least one titanium- and/or nickel-containing compound, then at least one compound containing an element of main group 5 of the Periodic Table and then butadiene are added.

10. A process as claimed in any one of claims 1 to 8,
wherein
firstly the solvent including polar component, then at least one titanium- and/or nickel-containing compound, then at least one organometallic compound, then at least one compound containing an element of main group 5 of the Periodic Table and then butadiene are added.

11. A process as claimed in any one of claims 1 to 10,
wherein
the organometallic compound is an organoaluminum compound.

12. A process as claimed in claim 11,
wherein
the organometallic compound is ethoxydiethylaluminum or ethylaluminum sesquichloride.

13. A process as claimed in either of claims 11 and 12,
wherein
the catalyst system contains nickel and the molar ratio of nickel to aluminum is from 1:3 to 1:6.

14. A process as claimed in either of claims 11 and 12,
wherein
the catalyst system contains titanium and the molar ratio of titanium to aluminum is from 1:10 to 1:60.

15. A process as claimed in any one of claims 1 to 14,
wherein
cycloocta-1,5-diene (COD) and/or vinylcyclohexene (VCH) are isolated.

16. A process as claimed in any one of the preceding claims,
wherein
the ratio of cyclododecatriene to higher oligomers is at least 10:1, preferably greater than 15:1, particularly preferably greater than 20:1.

17. A process as claimed in any one of the preceding claims,
wherein
the amount of chlorocyclododecatriene in the crude cyclododecatriene and/or in the purified cyclododecatriene is not more than 100 ppm, preferably 0.01-80 ppm, particularly preferably from 0.1 to 70 ppm, very particularly preferably from 1 to 50 ppm and from 1 to 30 ppm.

## Revendications

1. Procédé continu et/ou discontinu pour la préparation de cyclododécatriène par transformation de butadiène en présence d'un solvant, d'au moins un système catalytique contenant du nickel et/ou du titane ainsi que d'au moins un composé organométallique en un cyclododécatriène brut, **caractérisé en ce que**
- un composé contenant au moins un élément du 5ème groupe principal du système périodique des éléments est ajouté au système catalytique,
- le solvant contient, avant l'addition du composant catalytique, 10-1000 ppm d'un composant polaire de formule générale HO-R, R étant choisi dans le groupe constitué par C₁-C₁₈-alkyle ramifié et non ramifié, C₁-C₁₈-cycloalkyle, C₁-C₁₈-aryle, C₁-C₁₈-aralkyle et H, et
- la température de réaction, dans un système catalytique contenant du nickel, est d'au maximum 140°C et dans le cas d'un système catalytique contenant du titane, elle est d'au maximum 80°C.

2. Procédé selon la revendication 1, **caractérisé en ce que** le système catalytique contient de l'acétylacétonate de nickel.

3. Procédé selon la revendication 1, **caractérisé en ce que** le système catalytique contient du TiX₄, avec X = F, Cl, Br, I ou leurs mélanges.

4. Procédé selon l'une quelconque des revendications 1 à 3, **caractérisé en ce que** la réaction est réalisée, dans le cas d'un système catalytique contenant du nickel, à 60 jusqu'à 120°C et, dans le cas d'un système catalytique contenant du titane, à 30 jusqu'à 75°C.

5. Procédé selon l'une quelconque des revendications 1 à 4, **caractérisé en ce que** le composant contenant un élément du 5ème groupe principal du système périodique des éléments contient au moins un atome d'azote.

6. Procédé selon la revendication 5, **caractérisé en ce qu'**on utilise une amine primaire et/ou secondaire et/ou tertiaire et/ou de l'ammoniac et/ou une pyridine et/ou une pyridone.

7. Procédé selon l'une quelconque des revendications 1 à 6, **caractérisé en ce qu'**on utilise un solvant aromatique ou aliphatique ou un mélange de ceux-ci.

8. Procédé selon l'une quelconque des revendications 1 à 7, **caractérisé en ce que** le système catalytique présente une concentration dans le mélange réactionnel de 0,01 à 40 mmoles/l de nickel ou de titane.

9. Procédé selon l'une quelconque des revendications 1 à 8, **caractérisé en ce qu'**on ajoute d'abord le solvant, y compris le composant polaire, puis au moins un composé organométallique, puis au moins un composé contenant du titane et/ou du nickel, puis au moins un composé contenant un élément du 5ème groupe principal du système périodique des éléments puis du butadiène.

10. Procédé selon l'une quelconque des revendications 1 à 8, **caractérisé en ce qu'**on ajoute d'abord le solvant, y compris le composant polaire, puis au moins un composé contenant du titane et/ou du nickel, puis au moins un composé organométallique, puis au moins un composé contenant un élément du 5ème groupe principal du système périodique des éléments puis du butadiène.

11. Procédé selon l'une quelconque des revendications 1 à 10, **caractérisé en ce qu'**il s'agit, pour le composé organométallique, d'un composé organique de l'aluminium.

12. Procédé selon la revendication 11, **caractérisé en ce qu'**il s'agit, pour le composé organométallique, d'éthoxydiéthylaluminium ou de sesquichlorure d'éthylaluminium.

13. Procédé selon l'une quelconque des revendications 11 à 12, **caractérisé en ce que** le système catalytique contient du nickel et **en ce que** le rapport molaire entre le nickel et l'aluminium vaut 1:3 à 1:6.

14. Procédé selon l'une quelconque des revendications 11 à 12, **caractérisé en ce que** le système catalytique contient du titane et **en ce que** le rapport molaire entre le titane et l'aluminium vaut 1:10 à 1:60.

15. Procédé selon l'une quelconque des revendications 1 à 14, **caractérisé en ce qu'**on isole du cycloocta-1,5-diène (COD) et/ou du vinylcyclohexène (VCH).

16. Procédé selon l'une quelconque des revendications précédentes, **caractérisé en ce que** le rapport de cyclododécatriène aux oligomères supérieurs vaut au moins 10:1, est de préférence supérieur à 15:1, de manière particulièrement préférée supérieur à 20:1.

17. Procédé selon l'une quelconque des revendications précédentes, **caractérisé en ce que** la quantité de chlorocyclododécatriène dans le cyclododécatriène brut et/ou dans le cyclododécatriène purifié est d'au maximal 100 ppm, de préférence de 0,01-80 ppm, de manière particulièrement préférée de 0,1 à 70 ppm, de manière tout particulièrement préférée de 1 à 50 et 1 à 30 ppm.
